# FASCICULE DE BREVET EUROPEEN

(11) **EP 2 609 639 B1**
(45) Date de publication et mention de la délivrance du brevet: **05.10.2016**
(21) Numéro de dépôt: 11755148.1
(22) Date de dépôt: 23.08.2011
(51) Int. Cl.: H01L 51/54, C07C 251/22, G03G 5/06

(54) **UTILISATION DE MOLECULES A CARACTERE ZWITTERIONIQUE POUR LA FORMATION D'UNE COUCHE DE TRANSPORT DE TROUS OU D'ELECTRONS**
VERWENDUNG VON ZWITTERIONISCHEN MOLEKÜLEN ZUR HERSTELLUNG EINER LOCH- ODER EINER ELEKTRONENTRANSPORTSCHICHT
USE OF ZWITTERIONIC MOLECULES FOR FORMING A HOLE OR ELECTRON TRANSPORT LAYER

(30) Priorité: 24.08.2010 FR 1003428
(43) Date de publication de la demande: 03.07.2013
(73) Titulaire: Centre National de la Recherche Scientifique, 75016 Paris (FR); Université de Strasbourg, 67081 Strasbourg Cedex (FR); University Of Nebraska Lincoln, Lincoln, Nebraska 68508 (US)
(72) Inventeur: DOUDIN, Bernard, F-67000 Strasbourg (FR); BRAUNSTEIN, Pierre, F-67000 Strasbourg (FR); ROUTABOUL, Lucie, F-67000 Strasbourg (FR); DALMAS, Guillaume, F-67400 Illkirch-Graffenstaden (FR); ZHANG, Zhengzheng, Middleton, Wisconsin 53562 (US); DOWBEN, Peter, Trail Crete, Nebraska 68333 (US)
(74) Mandataire: Gevers & Orès
(86) Numéro de dépôt international: PCT/IB2011/053696
(87) Numéro de publication internationale: WO 2012/025878

(56) Documents cités:
- FR-A1- 2 877 337
- US-A1- 2005 025 994
- US-A1- 2005 211 292
- US-B1- 6 169 291
- Tibor Höltzl ET AL: "Chemical bonding in zwitterionic diamino- meta -quinonoids and their isomers", Journal of Physical Organic Chemistry, vol. 18, no. 11, 1 November 2005 (2005-11-01), pages 1123-1131, XP055131474, ISSN: 0894-3230, DOI: 10.1002/poc.978
- QING-ZHENG YANG ET AL: 'Tunable N-substitution in Zwitterionic Benzoquinonemonoimine Derivatives: Metal Coordination, Tandemlike Synthesis of Zwitterionic Metal Complexes, and Supramolecular Structures' CHEMISTRY - A EUROPEAN JOURNAL vol. 11, no. 24, 09 Décembre 2005, pages 7237 - 7246, XP055131486 DOI: 10.1002/chem.200500704 ISSN: 0947-6539

## Description

L'invention concerne l'utilisation de molécules à caractère zwitterionique pour la formation de couches de transport de trous ou d'électrons.

Elle concerne également un procédé de fabrication d'une couche d'un substrat ayant des propriétés de transport de trous ou d'électrons.

Elle concerne également un tel substrat et ses utilisations.

Les dispositifs électroniques de type organique représentent de nos jours un développement technologique majeur dans l'industrie des dispositifs électroniques.

Par exemple, les écrans à diode électroluminescente organique (OLED) ont récemment été mis sur le marché grand public.

Ils représentent une avancée considérable en termes de dispositifs électroniques flexibles, et de circuits conformationnels bon marché.

Cela entraîne une activité importante dans le domaine de la recherche fondamentale et appliquée sur les dispositifs électroniques organiques, dans le monde.

Les acteurs clés majeurs sont principalement en Asie, les plus importants étant des sociétés japonaises et coréennes.

En Europe, des développements directeurs sont établis.

Même si la majorité des dispositifs actuellement commercialisés sont obtenus en utilisant des techniques d'évaporation sous vide avec une sublimation de petites molécules, il existe toujours un besoin pour des solutions alternatives en utilisant des techniques chimiques humides en solution, ces techniques ayant été utilisées avec succès pour les dispositifs à base de polymères.

L'utilisation de telles techniques permettrait de surmonter les limitations dues au choix des molécules et ouvrirait la possibilité pour des applications à l'échelle industrielle, moins coûteuses.

L'électronique organique implique principalement l'utilisation de systèmes organiques semi-conducteurs pour créer des dispositifs mimant ceux, bien connus, utilisant la technologie du silicium, par exemple pour les diodes, les transistors, les dispositifs de conversion courant-lumière (lumière-émission), et les dispositifs à courant faible (pile photovoltaïque ou détecteur de lumière).

Les systèmes les plus importants sont :

### Les dispositifs organiques d'émission de lumière (OLED) :

Dans ces dispositifs un courant du porteur est transformé en lumière émise. La structure du dispositif est une séquence d'un empilement vertical de :
- une anode de type métallique (côté transparent),
- une couche de transport et d'injection de trous,
- une couche de recombinaison (émission),
- une couche de transport et d'injection d'électrons,
- une cathode de type métallique.

### Les dispositifs photovoltaïques organiques (OPV) :

La structure de ces dispositifs est similaire à celle des OLED, avec un choix différent de matériaux, maximisant l'efficacité de transformation des photons entrants en courant électrique.

### Les transistors organiques (OFET) :

Ces dispositifs sont conçus en géométrie latérale (ce qui explique le type FET de transistors envisagés).

Dans ces dispositifs, un film organique forme la couche conductrice active entre les électrodes source et drain métalliques.

Le contrôle de la grille est habituellement obtenu par un substrat de silicium dopé recouvert de SiO₂.

Pour tous ces systèmes, il est essentiel de s'assurer qu'un courant suffisant s'écoule.

Ceci requiert une barrière d'interface limitée entre les électrodes métalliques et la couche organique, ainsi qu'une bonne mobilité électronique des porteurs de charge dans les parties organiques.

Dans l'art, il est communément accepté qu'un des principaux goulots d'étranglement lors de l'utilisation pratique de tels dispositifs est la capacité limitée des contacts métalliques à injecter des trous dans les semi-conducteurs organiques, limitant ainsi l'écoulement du courant (et par exemple, la sortie de la lumière) du dispositif résultant.

Ceci est spécialement critique pour les dispositifs à émission de lumière.

L'injection de trous est habituellement obtenue en utilisant des matériaux à faible fonction de travail (par exemple LaB₆ et GdB₆) mais des matériaux d'électrode plus conventionnels pourraient être utilisés avec la bonne conception d'interfaces, par exemple AuCu₃, ITO (oxyde d'indium et d'étain), TiAu, AlAu.

Néanmoins, une barrière énergétique significative apparaît systématiquement avec la plupart des matériaux d'électrodes et cela est à surmonter.

La raison fondamentale clé est l'apparition de dipôles interfaciaux, résultants d'un déséquilibre métal-charge organique créant des dipôles de surface à l'interface.

Le champ électrique résultant augmente généralement la hauteur de la barrière pour injecter les trous dans un semi-conducteur organique, diminuant ainsi le transport des trous dans le dispositif.

Une autre propriété intrinsèque fortement désirable des conducteurs organiques est la possibilité de réaliser un bon conducteur porteur de charge d'électron (type n) car ils sont plus rares dans la nature.

L'invention vise à pallier les inconvénients des films semi-conducteurs organiques de l'art antérieur et à fournir des systèmes moléculaires de transport de trous ou d'électrons.

Elle permet aussi d'obtenir des interfaces moléculaires à propriétés électroniques améliorées.

A cet effet, l'invention propose l'utilisation de molécules à caractère zwitterionique ayant un fort dipôle intrinsèque, notamment ayant un moment dipolaire supérieur ou égal à 10 debyes, dans lesquelles il existe une délocalisation de charge au sein des parties anionique et cationique, pour la formation d'une couche de transport de trous ou d'électrons.

Les molécules connues à caractère zwitterionique sont caractérisées par le fait qu'aucune formule neutre ne peut les décrire. Il y a donc un découplage de charges résiduelles + et - (zwei ions), résultant en un dipôle électrique. Elles se rencontrent par exemple dans les domaines de la chimie organique ou organométallique.

Des exemples de molécules zwitterioniques organiques sont :

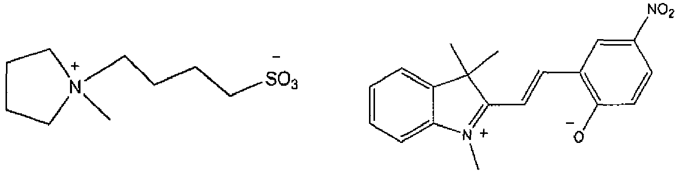

Des exemples de molécules zwitterioniques organométalliques sont :

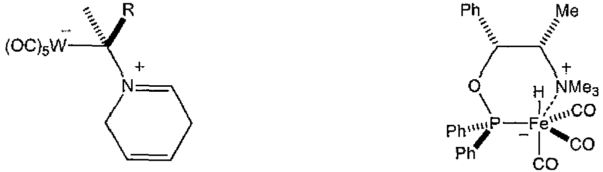

Les molécules à caractère zwitterionique sont des dérivés de *p*-benzoquinonemonoimines de formule I suivante : 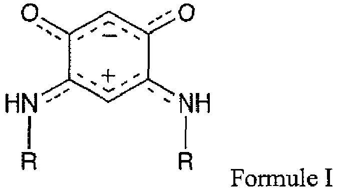 dans laquelle R est choisi parmi :
- H,
- un radical alkyle en C₁ à C₂₀, linéaire ou ramifié, pouvant être substitué par un ou plusieurs radicaux choisis parmi les radicaux hydroxy, amino, amino alkyle en C₁ à C₁₂, alkoxy en C₁ à C₁₂, pyridine, phosphine, thioether, thiol, alcène en C₁ à C₁₂, alcyne en C₁ à C₁₂ et halogène tel que F, I, Br et Cl,
- un radical benzyle pouvant être substitué par un ou plusieurs radicaux choisis parmi les radicaux hydroxy, amino, amino alkyle en C₁ à C₁₂, alkoxy en C₁ à C₁₂ et halogène, tel que F, I, Br et Cl.

Plus préférablement, on utilise des dérivés de formule I dans laquelle R est choisi parmi un atome d'hydrogène, ou un groupe -C₄H₉, -C₃H₆-S-CH₃, C₆H₅-CH₂- et -C₃H₆-O-CH₃.

Le plus préférablement, on utilise des dérivés de formule I dans laquelle R est choisi parmi un groupe -C₄H₉ et un groupe C₆H₅-CH₂-,

Selon un mode de réalisation, la couche de transport de trous et d'électrons formée par les molécules à caractère zwitterionique selon la formule joue le rôle d'une électrode conductrice.

L'invention propose également un procédé de fabrication d'un substrat ayant des propriétés de transport de trous ou d'électrons, caractérisé en ce qu'il comprend la liaison, sur au moins une surface d'un support, d'au moins un type de molécules à caractère zwitterionique selon la formule I.

La surface du support peut être une couche conductrice, notamment métallique (par exemple, Au), isolante (par exemple SiO₂, ou un oxyde métallique possédant des propriétés ferroélectriques, tel que LiNbO₃ ou un matériau possédant des terminaisons ayant une réactivité avec des oxydes) ou semiconductrice (par exemple SiOₓH_{y}), qui peut être obtenue en couche mince sur un support compatible avec les dispositifs de micro-électronique (par exemple, plaquette de Si recouverte de SiO₂).

Dans le procédé de l'invention, les molécules à caractère zwitterionique sont des dérivés de *p*-benzoquinoneimines de formule I suivante : 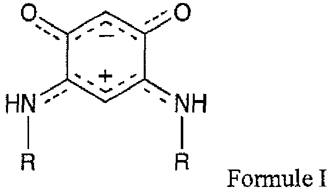 dans laquelle R est choisi parmi :
- H,
- un radical alkyle en C₁ à C₂₀, linéaire ou ramifié, pouvant être substitué par un ou plusieurs radicaux choisis parmi les radicaux hydroxy, amino, aminoalkyle en C₁ à C₁₂, alkoxy en C₁ à C₁₂, pyridine, phosphine, thioéther, thiol, alcène en C₁ à C₁₂, alcyne en C₁ à C₁₂ et halogène, tel que F, I, Br et Cl.
- un radical benzyle pouvant être substitué par un ou plusieurs radicaux choisis parmi les radicaux hydroxy, amino, amino alkyle en C₁ à C₁₂, alkoxy en C₁ à C₁₂, et halogène, tel que F, I, Br et Cl.

Mais, plus préférablement, les dérivés de formule I sont ceux dans lesquels R est choisi parmi un atome d'hydrogène, ou un groupe -C₄H₉, -C₃H₆-S-CH₃, C₆H₅-CH₂- et -C₃H₆-O-CH₃.

Le plus préférablement, les dérivés de formule I sont ceux dans lesquels R est choisi parmi un groupe -C₄H₉ et un groupe C₆H₅-CH₂-,

Egalement de préférence, dans le procédé de l'invention, le matériau constituant la surface jouant le rôle d'électrode, est choisi de préférence parmi Al, Au, AuCu₃, ITO, TiAu, AlAu, le graphène, LaB₆, GdB₆, Ni, Co, Fe, Pd, Pt, ainsi que les alliages de ces différents matériaux.

L'invention propose aussi un substrat ayant des propriétés de transport de trous ou d'électrons, caractérisé en ce qu'il comprend un support dont au moins une surface est en un matériau choisi parmi Al, Au, AuCu₃, ITO, TiAu, AlAu, le graphène, LaB₆, GdB₆, Ni, Co, Fe, Pd, Pt, ainsi que les alliages de ces différents matériaux, et en ce qu'une couche d'au moins un type de molécules à caractère zwitterionique selon la formule I-1 est liée à ladite surface.

La surface du support peut être une couche en un matériau conducteur tel qu'un métal, par exemple Au, ou en un matériau isolant tel que par exemple SiO₂ ou LiNbO₃, ou en un matériau semi-conducteur tel que par exemple SiOₓH_{y}, qui peut être obtenue en couche mince sur un support compatible avec les dispositifs de micro-électronique, par exemple une plaquette de Si recouverte de SiO₂.

Lorsque la surface du support est une couche en un matériau isolant ou semiconducteur, la couche d'au moins un type de molécules à caractère zwitterionique selon les formules I ou I-1, peut avantageusement jouer le rôle d'électrode conductrice. Dans ce cadre, les composés à caractère zwitterionique de formule (I) dans laquelle R est un groupe C₆H₅-CH₂- sont particulièrement préférés.

La couche d'au moins un type de molécules à caractère zwitterionique est formée de molécules de formule I-1 suivante : 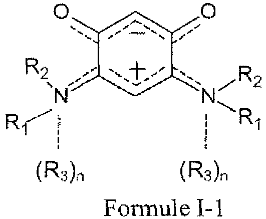 dans laquelle :
- n = 0 ou 1, et
- R₁ et R₂ sont choisis parmi les couples suivants :
   - lorsque R₁ est : 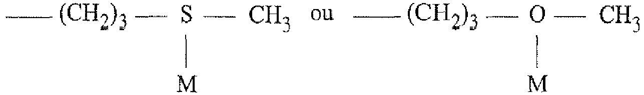 alors R2 est H et n=0
   - lorsque R₁ est : -C₄H₉ :
      ∘ soit R₂ est H₂ et n=1 auquel cas R₃ est M (interaction azote-métal)
      ∘ soit R₂ est H et n=1 auquel cas R₃ est M (interaction azote-métal avec déprotonation de la molécule sur la surface)
      ∘ soit R₂ est M et n=0,
   - lorsque R₁ est -CH₂-C₆H₅-M alors R₂ est H et n=0
   - lorsque R₁ est -CH₂-C₆H₅ :
      ∘ soit R₂ est H et n=1 auquel cas R₃ est M
      ∘ soit R₂ est M et n=0
dans lesquels M désigne un atome ou une molécule du matériau constituant la surface du support.

De préférence, R₁ est choisi parmi un groupe -CH₂-C₆H₅-M et un groupe :

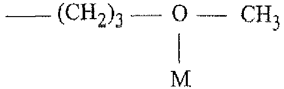

A titre illustratif, et sans vouloir être lié par une théorie particulière, lorsque la surface du support est une couche en un matériau conducteur, M peut être notamment un atome de métal, par exemple un atome Au. Lorsque cette surface est une couche en un matériau isolant, M peut être notamment un atome superficiel d'un oxyde métallique tel que LiNbO₃. Enfin, lorsque cette surface est une couche en un matériau semiconducteur tel que SiOₓH_{y}, M peut être un atome d'hydrogène (liaison hydrogène) ou oxygène.

La molécule zwitterionique de formule I-1 peut se lier avec M présent à la surface du support via des interactions avec des groupes riches en électrons présents dans sa structure, tels que des hétéroatomes possédant des doublets libres, notamment N, S ou O, ou encore via des groupements aromatiques (interactions π). Ces interactions s'apparentent généralement à un échange électronique entre la molécule zwitterionique et la surface du support (groupe M) et se caractérisent typiquement par un déplacement énergétique d'environ 0,5 eV.

L'invention propose encore un dispositif comprenant un substrat selon l'invention ou obtenu par le procédé de l'invention.

Dans un premier mode de réalisation, le dispositif est du type OLED.

Dans un second mode de réalisation, le dispositif est du type OPV.

Dans un troisième mode de réalisation, le dispositif est du type OFET.

L'invention sera mieux comprise et d'autres caractéristiques et avantages de celle-ci apparaîtront plus clairement à la lumière de la description explicative qui suit et en référence aux figures dans lesquelles :
- la figure 1 montre les spectres de photo-émission et inverse combinés des molécules zwitterioniques de l'invention,
- la figure 2 montre l'écoulement du courant à travers une molécule de benzoquinomonoimine (R= radical butyle C₄H₉) selon l'invention, en comparaison à la fuite de courant sans molécule, entre deux électrodes d'or séparées de 1 à 3 nanomètres,
- la figure 3 montre les courbes de sortie, c'est-à-dire les courbes de variations de l'intensité du courant entre l'électrode source et l'électrode drain en fonction de la tension appliquée à la grille d'un OFET selon l'art antérieur,
- la figure 4 montre les courbes de sortie, c'est-à-dire les courbes de variations de l'intensité du courant entre l'électrode source et l'électrode drain en fonction de la tension appliquée à la grille d'un OFET selon l'invention,
- la figure 5 représente la courbe de transfert, c'est-à-dire la courbe représentant la variation de l'intensité entre l'électrode source et l'électrode drain d'un OFET selon l'art antérieur, en fonction de la tension appliquée sur l'électrode grille de cet OFET ainsi que la courbe représentant la racine de cette variation d'intensité, et
- la figure 6 représente la courbe de transfert, c'est-à-dire la courbe de variation de l'intensité du courant entre l'électrode source et l'électrode drain d'un OFET selon l'invention, en fonction de la tension appliquée sur l'électrode grille de cet OFET ainsi que la courbe représentant la racine de cette variation.

L'invention porte sur l'utilisation de molécules à caractère zwitterionique selon la formule I pour former des couches de transport de trous ou d'électrons.

Ces molécules à caractère zwitterionique ont un dipôle intrinsèque significatif, notamment un moment dipolaire supérieur à 10 debyes, qui joue un rôle clé d'écran d'interface ainsi que d'ajustement du niveau HOMO (orbitale moléculaire occupée la plus élevée - "Highest Occupied Molecular Orbital") sur le bord de la bande de conduction d'un substrat en un matériau semi-conducteur.

En effet, les inventeurs ont découvert que l'utilisation de films (couches, surfaces) formés de molécules à caractère zwitterionique de la famille des p-benzoquinomonoimines diminue effectivement et efficacement la barrière d'interface, permettant aux électrons créés dans les films de s'échapper rapidement du substrat en matériau (semi-conducteur) et que les trous créés dans les films peuvent rapidement s'échapper du substrat en un matériau semi-conducteur et que de plus il est possible d'obtenir une densité non-nulle des états des films moléculaires au niveau de Fermi du système substrat en un matériau semi-conducteur / film de molécules métalliques.

On connaît de nombreuses familles de molécules à caractère zwitterionique.

On peut citer, à titre d'exemples, les molécules suivantes :

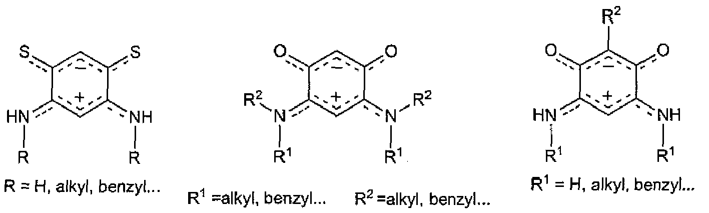

Cependant, une famille particulière de molécules à caractère zwitterionique sont les molécules de la famille des *p*-benzoquinonemonoimines de formule I suivante : 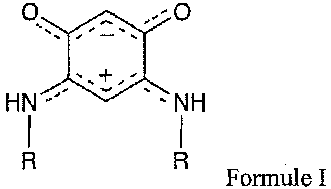 dans laquelle R représente :
- H,
- un radical alkyle en C₁ à C₂₀, linéaire ou ramifié, pouvant être substitué par un ou plusieurs radicaux choisis parmi les radicaux hydroxy, amino, amino alkyle en C₁ à C₁₂, alkoxy en C₁ à C₁₂, pyridine, phosphine, thioether, thiol, alcène en C₁ à C₁₂, alcyne en C₁ à C₁₂ et halogène, tel que F, I, Br et Cl.
- un radical benzyle pouvant être substitué par un ou plusieurs radicaux choisis parmi les radicaux hydroxy, amino, amino alkyle en C₁ à C₁₂, alkoxy en C₁ à C₁₂, et halogène, tel que F, I, Br et Cl.

La molécule de formule I archétype apparaît au schéma ci-après :

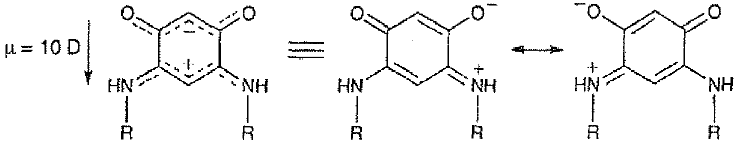

Ces petites molécules ont un dipôle électrique intrinsèque de 10 Debyes, qui est fort pour de si petites molécules.

L'azote peut être utilisé pour modifier le groupe d'extrémité R et ainsi moduler l'attachement à une surface.

Les composés de formule (I) peuvent être préparés par toute méthode connue de l'homme du métier et notamment selon le procédé décrit dans WO 2006/048547.

Par les termes "liés à la surface" ou "liaison à la surface" on entend, dans la présente invention, aussi bien un greffage qu'une adsorption. Plus précisément, il peut s'agir d'une liaison chimique mettant en commun des électrons de la molécule zwitterionique et du matériau constituant la surface du support, mais également d'interactions ou de liaisons plus faibles de types liaisons hydrogène, interaction de Van der Waals, ou de liaisons électrostatiques de type dipôles-dipôles.

Les groupes oxygène peuvent être utilisés pour des réactions de chimie de coordination, en utilisant un centre métallique pour lier deux molécules à caractère zwitterionique.

Ces molécules sont connues mais en tant que colorant (voir, par exemple, WO2006/048547).

Les expériences effectuées par les inventeurs sur ces molécules ont révélé qu'il était possible de lier ces molécules sur des surfaces et de caractériser leurs propriétés électroniques.

Ainsi ces molécules permettent de réaliser de nouveaux systèmes moléculaires liés à des surfaces.

Ces molécules organiques ont un dipôle intrinsèque faisant écran à l'effet nuisible du dipôle de l'interface métallique.

Il est possible avec ces molécules de déposer des films fins couvrant plus de 98 % de la surface d'un support en un matériau conducteur tel que l'or (Au), l'oxyde d'indium et d'étain (ITO), un alliage d'or et de cuivre (AuCu₃), un alliage de titane et d'or (TiAu) et un alliage d'aluminium et d'or (AlAu).

Il est également possible de déposer des films fins couvrant ces molécules couvrant des substrats peu ou non conducteurs comme la silice (SiO₂) ou le niobate de lithium (LiNbO₃), possédant également des propriétés ferroélectriques.

Les propriétés des films formés, en particulier, le fait que les électrons créés dans les films formés peuvent rapidement s'échapper du substrat et que les trous créés dans les films formés peuvent également s'échapper du substrat, montrent que ces molécules transportent très bien les porteurs de charge et ont des barrières d'interface limitées avec le substrat.

Ceci est extrêmement rare pour des molécules sur des surfaces.

Egalement, avec ces molécules il est possible d'obtenir une densité non-nulle des états des films moléculaires au niveau de Fermi du système substrat en un matériau métallique ou semi-conducteur ou isolant / film de molécules métalliques.

Cette propriété est remarquable car, à la connaissance des inventeurs, il n'existait pas jusqu'à présent de systèmes organiques dans lesquels le gap HOMO-LUMO (orbitale moléculaire non occupée la plus basse) correspond aussi étroitement au niveau de Fermi d'un substrat conducteur ou semiconducteur.

Sans vouloir être liés par la théorie, les inventeurs attribuent cette propriété exceptionnelle, en utilisant leurs connaissances des orientations moléculaires, au dipôle intrinsèque des molécules utilisées dans l'invention.

L'absence de déplacement résultant dans les spectres de photo-émission est un résultat immédiat de la conductivité significative des films moléculaires ainsi formés dont le seul autre exemple connu des hommes de l'art était le graphite.

Afin de mieux faire comprendre l'invention, on va maintenant en décrire, à titre purement illustratif et non limitatif, plusieurs exemples de mise en oeuvre.

Dans les exemples qui suivent, on a adsorbé des dérivés de p-benzoquinonemonoimines de formule I sur des substrats ayant une surface en or.

La surface d'or, préalablement lavée en séquence d'exposition à l'éthanol (en bain ultra-sons) et acétone, est laissée pendant 16h dans une solution des molécules. Généralement la solution a une concentration de 0,8 mol/1, le solvant utilisé étant principalement le dichlorométhane. La surface est lavée plusieurs fois avec de l'éthanol puis séchée et conservée sous atmosphère inerte.

L'épaisseur du film obtenu est comprise entre 0,5 et 1 nm inclus, après un lavage intensif (10 minutes sous flux maintenu d'éthanol).

### Exemple 1

On a utilisé une molécule de formule I dans laquelle R est H.

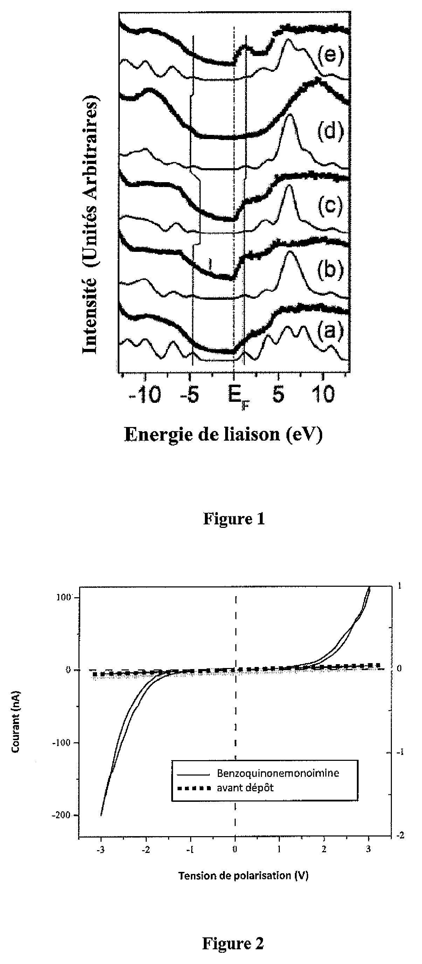

Les spectres de photo-émission inverse et de photo-émission combinés sont notés (a) en figure 1.

La structure schématique de la molécule indiquant la structure lorsque adsorbée sur l'or ou autrement liée au support en or est une combinaison des deux structures suivantes :

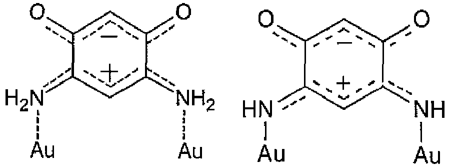

### Exemple 2

On a utilisé un dérivé de *p*-benzoquinonemonoimines de formule I dans laquelle R est C₄H₉.

Les spectres de photo-émission inverse et de photo-émission combinée du film obtenu sont montrés en figure 1 où ils sont notés (b).

La structure du composé de formule I utilisé lorsqu'adsorbé sur l'or ou lié à la surface en or d'un support est une combinaison des structures suivantes :

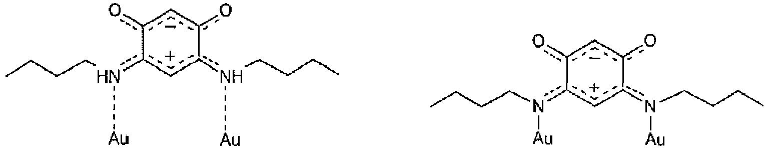

### Exemple 3

On a utilisé un dérivé de *p*-benzoquinonemonoimines de formule I dans lequel R est C₃H₆-S-CH₃.

Les spectres de photo-émission inverse et de photo-émission combinée avec le film ainsi formé est montré en figure 1 où ils sont notés (c).

La structure des molécules utilisées lorsqu'adsorbées à la surface du support en or est la suivante :

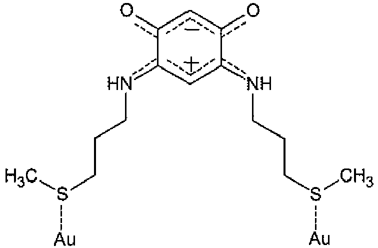

### Exemple 4

On a utilisé un dérivé de *p*-benzoquinonemonoimines de formule I dans lequel R est C₃H₆-O-CH₃.

Les spectres de photo-émission inverse et de photo-émission combinée sont représentés en figure 1 où ils sont notés (d).

La structure de ces molécules lorsqu'adsorbées sur la surface d'un support en or est la suivante:

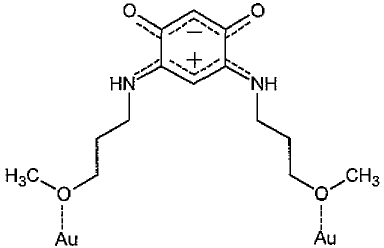

### Exemple 5

On a utilisé un dérivé de *p*-benzoquinonemonoimines de formule I dans lequel R est CH₂-C₆H₅.

Les spectres de photo-émission inverse et de photo-émission combinés des films obtenus avec ce dérivé sont montrés en figure 1 où ils sont notés (e).

La structure de ce dérivé lorsqu'adsorbé sur la surface d'un support en or est une combinaison des structures suivantes :

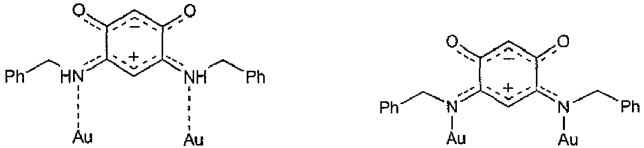

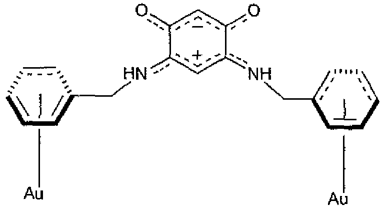

Dans la figure 1, les spectres de photo-émission inverse et de photo-émission combinés obtenus avec le film selon l'invention sont représentés en traits gras alors que la densité théorique obtenue par des modèles de calcul d'une seule molécule correspondante montrés pour comparaison est représentée par une ligne fine.

### Exemple 6

Le courant s'écoulant à travers un dérivé de p-benzoquinonemonoimines de formule I dans laquelle R est C₄H₉ en fonction de la tension, a également été mesuré en comparaison au courant de fuite sans ce dérivé, entre deux électrodes d'or séparées de 1-3 nm.

Ces courbes sont représentées en figure 2.

### Exemple 7

Le travail de sortie du dispositif de l'exemple 2 a été mesuré ainsi que celui d'un dispositif identique à celui de l'exemple 2 mais sur lequel est greffé une molécule de formule I dans laquelle R est un groupement éthyle, ceci pour vérifier que la présence des composés de formule I permet d'abaisser le travail de sortie de façon reproductible et stable dans le temps.

Pour cela, on a procédé à une mesure, par spectroscopie de photoélectrons U.V (UPS), à pression ambiante, des électrons émis par ces dispositifs.

Par UPS on mesure tous les électrons émis par le matériau, incluant les électrons secondaires.

Le travail de sortie correspond au début de détection des électrons photo-émis.

A titre de référence, on a mesuré le travail de sortie, dans les mêmes conditions d'un substrat en or identique à celui utilisé pour fabriquer les échantillons utilisés aux exemples qui précèdent et traité de la même façon mais sur lequel aucune molécule n'était greffée.

Le travail de sortie de l'échantillon de référence en or est de 5,13 eV, le travail de sortie du dispositif de l'exemple 2 est de 4,66 eV et le travail de sortie du dispositif dans lequel le dérivé de *p*-benzoquinonemonoimines de formule I dans lequel R est un groupement éthyle est compris entre 4,72 eV et 4,80 eV.

Ces mesures ont été effectuées immédiatement après la fabrication du dispositif.

Ainsi, la différence entre le travail de sortie de l'échantillon de référence, 5,13 eV, et ceux des films minces d'or sur lesquels les molécules de formule I de l'invention ont été greffés est d'environ 0,3 à 0,4 eV, et ce de façon reproductible. Cela montre qu'effectivement les molécules de l'invention permettent d'abaisser le travail de sortie et montrent bien l'effet d'écrantage de dipôle d'interface de ces molécules.

L'échantillon de référence et l'échantillon de l'exemple 2 ont été laissés quatre jours à atmosphère ambiante, c'est-à-dire à l'air et à l'humidité ambiants.

Le travail de sortie a à nouveau été mesuré.

Le travail de sortie de l'échantillon de référence n'avait pas changé (il était toujours de 5,13 eV après ces quatre jours) et le travail de sortie de l'échantillon de l'exemple 2 était de 4,72 eV.

Cela montre bien que les molécules de formule I lorsque greffées sur une surface en or, restent stables, c'est-à-dire ne se dégradent pas et restent greffées.

### Résultats

On voit à partir de la figure 2 que le dérivé de *p*-benzoquinonemonoimines de formule I dans laquelle R est un groupe butyle (-C₄H₉) a une excellente densité d'empilement, un recouvrement parfait d'un substrat métallique lorsque des couches ultra-minces sont obtenues, et montre des propriétés de conductions électrique entre deux électrodes métalliques.

Pour ces raisons, ce dérivé de *p*-benzoquinonemonoimines est un composé préféré pour l'utilisation dans l'invention.

On voit à partir de la figure 1 que le dérivé de *p-*benzoquinonemonoimine avec le niveau le plus élevé de mobilité électronique est le composé de formule I dans lequel R est un groupe benzyle.

De plus, avec ce composé, aucun effet de chargement électrostatique lors d'une irradiation de photons à long terme n'a été trouvé.

Pour cette raison, ce dérivé est particulièrement préféré pour l'utilisation dans l'invention.

On voit à partir de ce qui précède qu'un autre objet de l'invention est un procédé de fabrication d'un substrat ayant des propriétés de transport de trous ou d'électrons qui comprend la liaison sur au moins une surface en un matériau de préférence conducteur d'un support, d'au moins un type de molécules à caractère zwitterionique.

Dans le procédé de l'invention, les molécules à caractère zwitterionique sont des dérivés de *p*-benzoquinoneimines de formule I suivante : 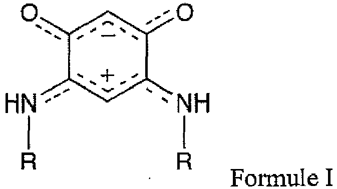 dans laquelle R représente :
- H,
- un radical alkyle en C₁ à C₂₀, linéaire ou ramifié, pouvant être substitué par un ou plusieurs radicaux choisis parmi les radicaux hydroxy, amino, amino alkyle en C₁ à C₁₂, alkoxy en C₁ à C₁₂, pyridine, phosphine, thioether, thiol, alcène en C₁ à C₁₂, alcyne en C₁ à C₁₂ et halogène, tel que F, I, Br et Cl.
- un radical benzyle pouvant être substitué par un ou plusieurs radicaux choisis parmi les radicaux hydroxy, amino, amino alkyle en C₁ à C₁₂, alkoxy en C₁ à C₁₂ et halogène, tel que F, I, Br et Cl.

De préférence, dans la formule I, R est choisi parmi un atome d'hydrogène, ou un groupe -C₄H₉, -C₃H₆-S-CH₃, C₆H₅-CH₂- et -C₃H₆-O-CH₃.

Le plus préférablement, la formule I, R est choisi parmi un groupe -C₄H₉ et un groupe C₆H₅-CH₂-.

Quant au matériau, il est choisi de préférence parmi Al, Au, AuCu₃, ITO, TiAu, AlAu, le graphène, LaB₆, GdB₆, Ni, Co, Fe, Pd, Pt, ainsi que les alliages de ces différents matériaux.

Par ce procédé et en utilisant ces molécules à caractère zwitterionique, on obtient un substrat ayant des propriétés de transport de trous ou d'électrons.

Ce substrat est également un objet de l'invention.

Il comprend un support dont au moins une surface est en un matériau choisi parmi Al, Au, AuCu₃, ITO, TiAu, AlAu, le graphène, LaB₆, GdB₆, Ni, Co, Fe, Pd, Pt, ainsi que les alliages possibles de ces différents matériaux, une couche d'au moins un type de molécules à caractère zwitterionique de la famille des p-benzoquinomonoimines étant liée à ladite surface.

De préférence, la couche d'au moins un type de molécules à caractère zwitterionique est formée de molécules de formule I-1 suivante : 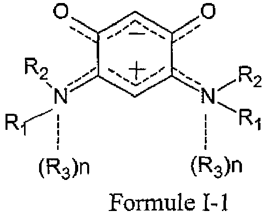 dans laquelle :
- n = ou 1, et
- R₁ et R₂ sont choisis parmi les couples suivants,
   - lorsque R₁ est : 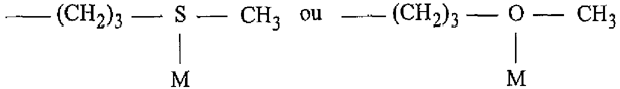 alors R₂ est H et n=0
   - lorsque R₁ est -C₄H₉ :
      ∘ soit R₂ est H₂ et n=1 auquel cas R₃ est M (interaction azote-métal)
      ∘ soit R₂ est H et n=1 auquel cas R₃ est M (interaction azote-métal avec déprotonation de la molécule sur la surface)
      ∘ soit R₂ est M et n=0,
   - lorsque R₁ est -CH₂-C₆H₅-M alors R₂ est H et n=0
   - lorsque R₁ est -CH₂-C₆H₅ :
      ∘ soit R₂ est H et n=1 auquel cas R₃ est M (intéraction azote-métal)
      ∘ soit R₂ est M et n=0 (déprotonation de la molécule sur la surface)
dans lesquels M désigne un atome ou une molécule du matériau constituant la surface du support.

Ce substrat a été utilisé avec succès en tant qu'écran de barrière d'interface d'un système électrode / film moléculaire, en tant que couche interfaciale pour effet diode et en tant que couche optiquement transparente ayant des propriétés conductrices.

Il a ainsi été intégré dans un dispositif tel qu'un dispositif du type OLED, du type OPV et du type OFET.

Ces utilisations et dispositifs sont également des objets de l'invention.

### Exemple 8 (comparatif)

On a fabriqué un dispositif OPV de l'art antérieur.

Pour cela on a recouvert la surface d'un substrat en verre d'une couche d'ITO.

Sur la surface libre de cette couche d'ITO, on a déposé une couche de poly(3,4-éthylènedioxythiophène)(polystyrène sulfonate)(PEDOT-PSS).

Sur la surface libre de cette couche PEDOT-PSS, on a déposé une couche organique composée d'un mélange à un rapport en masse de 1:1 de poly 3-hexylthiophène (P3HT) et de phényle C61-acide butyrique méthyl ester (PCBM).

Cette couche organique est ensuite revêtue d'une couche d'aluminium.

Cette configuration de dispositif OPV est l'archétype des meilleures cellules photovoltaïques organiques connues à ce jour.

Le rendement mesuré de cette cellule est de 2,47625.

La densité de courant moyenne de cette cellule est de 7,512857 milliampères/cm².

Ce type de cellule a pourtant un inconvénient : la couche d'ITO peut être dégradée, dans le temps par le PEDOT-PSS.

### Exemple 9

On a fabriqué une cellule de type OPV de la même façon qu'à l'exemple 8 mais, avant de déposer la couche de PEDOT-PSS, on a greffé des molécules zwitterioniques selon l'invention de formule I dans laquelle R est un groupement butyle.

Le rendement de cette cellule est de 2,62625.

La densité de courant moyenne de cette cellule est de 7,972 milliampères/cm².

On voit à partir des exemples 8 et 9 que le greffage des molécules de formule I selon l'invention permet d'améliorer l'ensemble des propriétés d'une cellule OPV aussi bien en termes de rendement que de densité de courant et de plus permet de protéger la couche d'ITO de l'effet de dégradation de la couche de PEDOT-PSS.

### Exemple 10 (comparatif)

On a fabriqué un transistor organique à film mince de type OFET.

Pour cela, on a déposé, sur un substrat en silicium dopé p (qui constitue la grille du transistor), une couche d'un matériau diélectrique (ici SiO₂).

Sur la couche de matériau diélectrique on a déposé deux électrodes, une électrode source et une électrode drain, et on a recouvert le matériau diélectrique et les électrodes source et drain avec une couche rendant le substrat hydrophobe, dans cet exemple, une couche d'hexaméthyldisilazane (HMDS).

Enfin, on a déposé une couche active de P3HT.

On a mesuré la variation de l'intensité du courant circulant entre les électrodes source et drain, notée I_{DS} en fonction de la tension appliquée sur la grille.

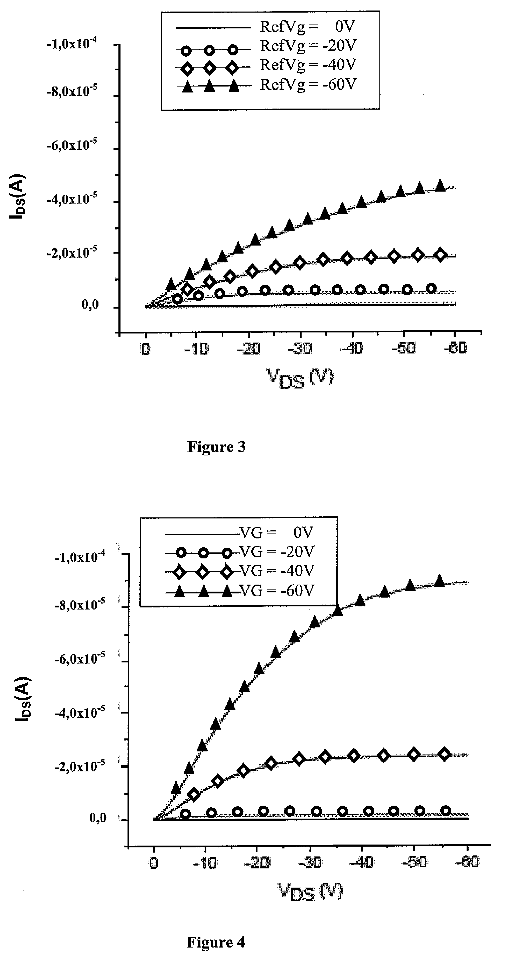

Les résultats sont représentés en figure 3 (courbe de sortie) et en figure 4 (courbe de transfert).

A partir des résultats de la courbe de sortie, la mobilité du canal semi-conducteur a été déduite.

A partir des résultats de la courbe de transfert, le rapport ION/IOFF a été déterminé.

Les résultats pour trois échantillons testés et fabriqués dans les mêmes conditions sont reportés au tableau 1 ci-après :

**Tableau 1**

| **Echantillon** | µₛₐₜ (cm²/V.s) | ION/IOFF |
|---|---|---|
| 1 | 3.7.10⁻³ | 1.8.10⁺² |
| 2 | 3.13.10⁻³ | 4.8.10⁺² |
| 3 | 3.6.10⁻³ | 5.3.10⁺² |

On voit à partir du tableau 1 que la mobilité µₛₐₜ varie entre 3,13 10⁻³ cm²/V.s et 3,7 10⁻³ cm²/V.s.

Le rapport ION/IOFF, qui est représentatif du rapport d'interruption du transistor fabriqué, varie, lui, entre 1,8 10⁻² et 5,3 10⁻².

On constate à partir de la figure 3 que le courant I_{DS} maximum est obtenu pour une tension appliquée à la grille de -60 V et reste inférieur à -4,0 x 10⁻⁵ A.

### Exemple 11

On a fabriqué un dispositif OFET de la même façon qu'à l'exemple 10, sauf que l'on a greffé des molécules zwitterioniques selon l'invention ayant la formule I dans laquelle R est un groupement butyle sur l'ensemble du matériau diélectrique et des électrodes source et drain, avant le dépôt de la couche de semi-conducteur organique.

Il est normalement contre-indiqué de déposer des molécules porteuses de charge sur le matériau diélectrique car cela diminue ses propriétés d'interface avec le canal semi-conducteur.

Mais dans cet exemple, malgré l'inconvénient de déposer des molécules porteuses de charge sur le matériau diélectrique, on a obtenu un dispositif dont les propriétés en termes de mobilité des électrons, de rapport ION/IOFF, sont améliorées.

En effet, on a mesuré la mobilité et le rapport ION/IOFF de ce dispositif.

Les résultats obtenus sur trois échantillons testés et fabriqués dans les mêmes conditions sont reportés au tableau 2 ci-après :

**Tableau 2**

| **Echantillon** | *µ*ₛₐₜ (cm²/V.s) | ION/IOFF |
|---|---|---|
| 1 | 5.3.10⁻³ | 4.1.10⁺⁴ |
| 2 | 1.2.10⁻³ | 2.04.10⁺⁴ |
| 3 | 1.4.10⁻² | 1.05.10⁺⁵ |

Comme on le voit à partir du tableau 2, la mobilité varie entre 1,2 10⁻³ et 1,4 10⁻² cm²/V.s et le rapport ION/IOFF varie entre 2,04 10⁴ et 1,05 10⁵, ce qui représente une amélioration notable par rapport à la cellule OFET de l'art antérieur.

De plus, on a mesuré le courant I_{DS} en fonction de la tension appliquée sur la grille, pour l'OFET obtenu à l'exemple 10 et pour l'OFET obtenu à l'exemple 11.

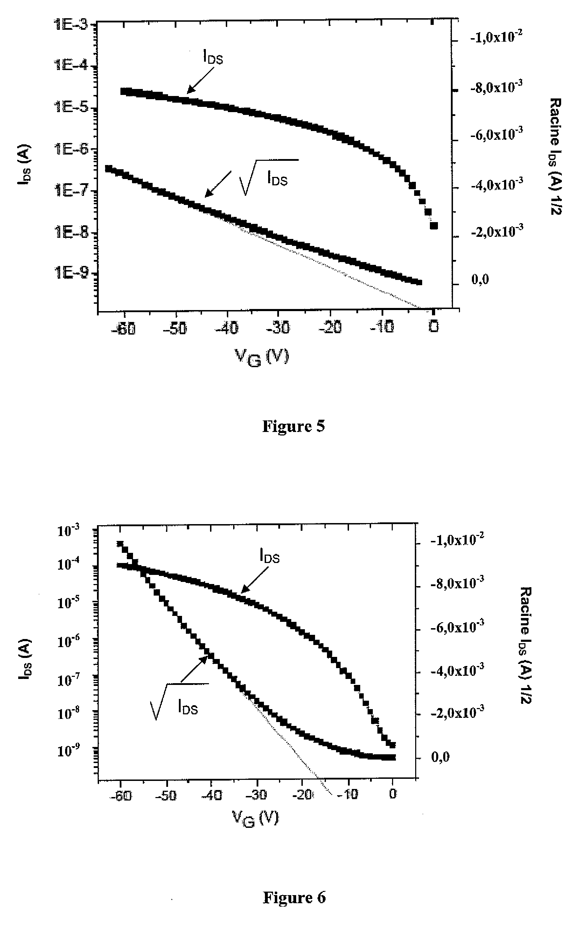

Les résultats sont représentés en figures 5 et 6.

Comme on le voit en comparant les figures 5 et 6, le courant circulant entre les électrodes est bien plus élevé dans le cas du dispositif OFET selon l'invention : il atteint un maximum supérieur à - 8,0 10⁵ pour une tension de grille de 60 V.

Ainsi, en utilisant les molécules de formule I de l'invention, la mobilité des OFET est augmentée d'un facteur de 2 et le rapport ION/IOFF est augmenté de près de 2 ordres de grandeur.

Un avantage particulier est le courant au repos, c'est-à-dire le courant lorsque les tensions entre les électrodes source et drain, ainsi que les tensions de grille sont toutes nulles. Les dispositifs selon l'invention montrent systématiquement un courant plus faible, ce qui indique un meilleur comportement ohmique à faible potentiel appliqué, dû au meilleur alignement des niveaux énergétiques à l'interface métal-semi-conducteur organique.

Un courant résiduel plus faible entre les électrodes source et drain est avantageux en termes de pertes énergétiques lorsque le transistor n'est pas actif.

Un courant à saturation entre les électrodes source et drain plus élevé est avantageux en termes d'applications de puissance du transistor.

## Revendications

1. Utilisation de dérivés de *p*-benzoquinonemonoimines de formule I suivante : 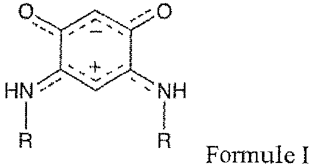 dans laquelle R représente :
- H,
- un radical alkyle en C₁ à C₂₀, linéaire ou ramifié, pouvant être substitué par un ou plusieurs radicaux choisis parmi les radicaux hydroxy, amino, amino alkyle en C₁ à C₁₂, alkoxy en C₁ à C₁₂, pyridine, phosphine, thioether, thiol, alcène en C₁ à C₁₂, alcyne en C₁ à C₁₂ et halogène,
- un radical benzyle pouvant être substitué par un ou plusieurs radicaux choisis parmi les radicaux hydroxy, amino, amino alkyle en C₁ à C₁₂, alkoxy en C₁ à C₁₂ et halogène,
pour la formation d'une couche de transport de trous ou d'électrons.

2. Utilisation selon la revendication 1, **caractérisée en ce que** dans la formule I, R est choisi parmi un atome d'hydrogène, ou un groupe -C₄H₉, -C₃H₆-S-CH₃, C₆H₅-CH₂- et -C₃H₆-O-CH₃.

3. Utilisation selon la revendication 1 ou 2 **caractérisée en ce que** dans la formule I R est choisi parmi un groupe -C₄H₉ et un groupe C₆H₅-CH₂-,

4. Procédé de fabrication d'un substrat ayant des propriétés de transport de trous ou d'électrons **caractérisé en ce qu'**il comprend la liaison, sur au moins une surface en un matériau conducteur d'un support, d'au moins un dérivé de *p-*benzoquinoneimines de formule I suivante : 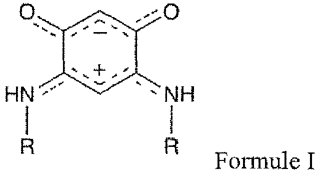 dans laquelle R représente :
- H,
- un radical alkyle en C₁ à C₂₀, linéaire ou ramifié, pouvant être substitué par un ou plusieurs radicaux choisis parmi les radicaux hydroxy, amino, amino alkyle en C₁ à C₁₂, alkoxy en C₁ à C₁₂, pyridine, phosphine, thioether, thiol, alcène en C₁ à C₁₂, alcyne en C₁ à C₁₂ et halogène,
- un radical benzyle pouvant être substitué par un ou plusieurs radicaux choisis parmi les radicaux hydroxy, amino, amino alkyle en C₁ à C₁₂, alkoxy en C₁ à C₁₂ et halogène.

5. Procédé selon la revendication 4, **caractérisé en ce que** dans la formule I, R est choisi parmi un atome d'hydrogène, ou un groupe -C₄H₉, -C₃H₆-S-CH₃, C₆H₅-CH₂- et -C₃H₆-O-CH₃.

6. Procédé selon la revendication 4 ou 5 **caractérisé en ce que** dans la formule I R est choisi parmi un groupe -C₄H₉ et un groupe C₆H₅-CH₂-.

7. Procédé selon l'une quelconque des revendications 4 à 6, **caractérisé en ce que** la surface est en un matériau choisi parmi Al, Au, AuCu₃, ITO, TiAu, AlAu, le graphène, LaB₆, GdB₆, Ni, Co, Fe, Pd, Pt, ainsi que les alliages de ces différents matériaux.

8. Substrat ayant des propriétés de transport de trous ou d'électrons, **caractérisé en ce qu'**il comprend un support dont au moins une surface est en un matériau choisi parmi Al, Au, AuCu₃, ITO, TiAu, AlAu, le graphène, LaB₆, GdB₆, Ni, Co, Fe, Pd, Pt, ainsi que les alliages de ces différents matériaux et **en ce qu'**une couche d'au moins un type de molécules de formule I-1 suivante : 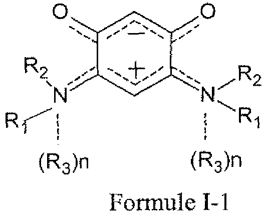 dans laquelle :
- n = 0 ou 1, et
- R₁ et R₂ sont choisis parmi les couples suivants,
·R₁ est 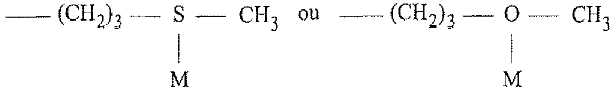 auquel cas R₂ est H et n=0
· R₁ est : -C₄H₉, auquel cas :
∘ soit R₂ est H et n=1 auquel cas R₃ est M
∘ soit R₂ est M et n=0
· R₁ est -CH₂-C₆H₅-M auquel cas R₂ est H et n=0
· R₁ est -CH₂-C₆H₅, auquel cas :
∘ soit R₂ est H et n=1 auquel cas R₃ est M
∘ soit R₂ est M et n=0
dans lesquels M désigne un atome ou une molécule du matériau dont est constitué ladite surface du support,
est liée à ladite surface.

9. Substrat selon la revendication 8 **caractérisé en ce que** dans la formule I-1, R₁ est choisi parmi un groupe -CH₂-C₆H₅-M et un groupe : 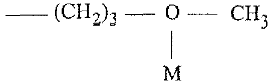 dans lesquels M désigne un atome ou une molécule du matériau dont est constitué ladite surface du support.

10. Dispositif comprenant un substrat ayant des propriétés de transport de de trous ou d'électrons **caractérisé en ce que** le substrat comprend un support dont au moins une surface est en un matériau conducteur et ce qu'une couche d'au moins un dérivé de *p*-benzoquinoneimines de formule I suivante : 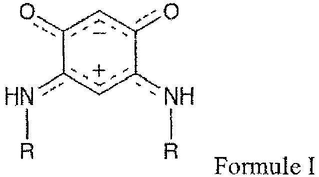 dans laquelle R représente :
- H,
- un radical alkyle en C₁ à C₂₀, linéaire ou ramifié, pouvant être substitué par un ou plusieurs radicaux choisis parmi les radicaux hydroxy, amino, amino alkyle en C₁ à C₁₂, alkoxy en C₁ à C₁₂, pyridine, phosphine, thioether, thiol, alcène en C₁ à C₁₂, alcyne en C₁ à C₁₂ et halogène,
- un radical benzyle pouvant être substitué par un ou plusieurs radicaux choisis parmi les radicaux hydroxy, amino, amino alkyle en C₁ à C₁₂, alkoxy en C₁ à C₁₂ et halogène,
est liée à ce matériau conducteur.

11. Dispositif selon la revendication 10, **caractérisé en ce que** dans la formule I, R est choisi parmi un atome d'hydrogène, ou un groupe -C₄H₉, -C₃H₆-S-CH₃, C₆H₅-CH₂- et -C₃H₆-O-CH₃.

12. Dispositif selon la revendication 10 ou 11, **caractérisé en ce que** dans la formule I, R est choisi parmi un groupe -C₄H₉ et un groupe C₆H₅-CH₂-.

13. Dispositif selon l'une quelconque des revendications 10 à 12, **caractérisé en ce que** le matériau conducteur est en un matériau choisi parmi Al, Au, AuCu₃, ITO, TiAu, AlAu, le graphène, LaB₆, GdB₆, Ni, Co, Fe, Pd, Pt, ainsi que les alliages de ces différents matériaux.

14. Dispositif selon la revendication 10, **caractérisé en ce que** le matériau conducteur est choisi parmi Al, Au, AuCu₃, ITO, TiAu, AlAu, le graphène, LaB₆, GdB₆, Ni, Co, Fe, Pd, Pt, ainsi que les alliages de ces différents matériaux, et **en ce que** le au moins un dérivé de *p*-benzoquinoneimines est une molécule de formule I-1 suivante : 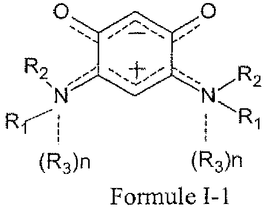 dans laquelle :
- n = 0 ou 1, et
- R₁ et R₂ sont choisis parmi les couples suivants,
. R₁ est 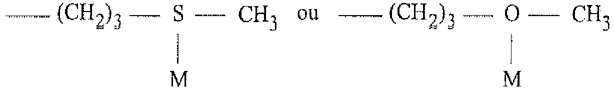 auquel cas R₂ est H et n=0
. R₁ est : -C₄H₉, auquel cas :
∘ soit R₂ est H et n=1 auquel cas est M
∘ soit R₂ est M et n=0
. R₁ est -CH₂-C₆H₅-M auquel cas R₂ est H et n=0
. R₁ est -CH₂-C₆H₅, auquel cas :
o soit R₂ est H et n=1 auquel cas R₃ est M
∘ soit R₂ est M et n=0
dans lesquels M désigne un atome ou une molécule du matériau conducteur..

15. Dispositif selon la revendication 14, caractérisé en ce dans la formule I-1, R₁ est choisi parmi un groupe -CH₂-C₆H₅-M et un groupe : 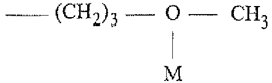 dans lesquels M désigne un atome ou une molécule du matériau conducteur.

16. Dispositif selon l'une quelconque des revendications 10 à 15, **caractérisé en ce qu'**il s'agit d'un dispositif du type OLED.

17. Dispositif selon l'une quelconque des revendications 10 à 15, **caractérisé en ce qu'**il s'agit d'un dispositif du type OPV.

18. Dispositif selon l'une quelconque des revendications 10 à 12, **caractérisé en ce qu'**il s'agit d'un dispositif du type OFET.

## Patentansprüche

1. Verwendung von p-Benzochinonmonoimin-Derivaten der folgenden Formel I: 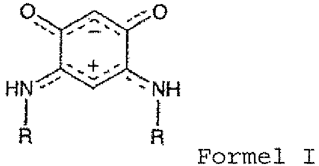 in der R darstellt:
- H,
- einen linearen oder verzweigten C₁-C₂₀-Alkylrest, der mit einem Rest oder mehreren Resten, ausgewählt aus den Resten Hydroxy, Amino, C₁-C₁₂-Alkylamino, C₁-C₁₂-Alkoxy, Pyridin, Phosphin, Thioether, Thiol, C₁-C₁₂-Alken, C₁-C₁₂-Alkin und Halogen, substituiert sein kann,
- einen Benzylrest, der mit einem Rest oder mehreren Resten, ausgewählt aus den Resten Hydroxy, Amino, C₁-C₁₂-Aminoalkyl, C₁-C₁₂-Alkoxy und Halogen, substituiert sein kann, für die Bildung einer Löcher- oder Elektronentransportschicht.

2. Verwendung gemäß Anspruch 1, **dadurch gekennzeichnet, dass** in der Formel I R aus einem Wasserstoffatom oder einer Gruppe -C₄H₉, -C₃H₆-S-CH₃, C₆H₅-CH₂- und -C₃H₆-O-CH₃ ausgewählt ist.

3. Verwendung gemäß Anspruch 1 oder 2, **dadurch gekennzeichnet, dass** in der Formel I R aus einer Gruppe -C₄H₉ und einer Gruppe C₆H₅-CH₂- ausgewählt ist.

4. Verfahren zur Herstellung eines Substrats, das Löcher- oder Elektronentransporteigenschaften hat, **dadurch gekennzeichnet, dass** es auf wenigstens einer Oberfläche aus einem leitenden Material eines Trägers die Bindung wenigstens eines p-Benzochinonimin-Derivats der folgenden Formel I umfasst: 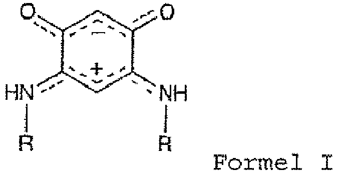 in der R darstellt:
- H,
- einen linearen oder verzweigten C₁-C₂₀-Alkylrest, der mit einem Rest oder mehreren Resten, ausgewählt aus den Resten Hydroxy, Amino, C₁-C₁₂-Alkylamino, C₁-C₁₂-Alkoxy, Pyridin, Phosphin, Thioether, Thiol, C₁-C₁₂-Alken, C₁-C₁₂-Alkin und Halogen, substituiert sein kann,
- einen Benzylrest, der mit einem Rest oder mehreren Resten, ausgewählt aus den Resten Hydroxy, Amino, C₁-C₁₂-Aminoalkyl, C₁-C₁₂-Alkoxy und Halogen, substituiert sein kann.

5. Verfahren gemäß Anspruch 4, **dadurch gekennzeichnet, dass** in der Formel I R ausgewählt ist aus einem Wasserstoffatom oder einer Gruppe -C₄H₉, -C₃H₆-S-CH₃, C₆H₅-CH₂- und -C₃H₆-O-CH₃.

6. Verfahren nach Anspruch 4 oder 5, **dadurch gekennzeichnet, dass** in der Formel I R aus einer Gruppe -C₄H₉ und einer Gruppe C₆H₅-CH₂- ausgewählt ist.

7. Verfahren gemäß einem der Ansprüche 4 bis 6, **dadurch gekennzeichnet, dass** die Oberfläche aus einem Material ist, das ausgewählt ist aus Al, Au, AuCu₃, ITO, TiAu, AlAu, Graphen, LaB₆, GdB₆, Ni, CO, Fe, Pd, Pt sowie den Legierungen dieser unterschiedlichen Materialien.

8. Substrat, das Löcher- oder Elektronentransporteigenschaften hat, **dadurch gekennzeichnet, dass** es einen Träger umfasst, von dem wenigstens eine Oberfläche aus einem Material ist, das ausgewählt ist aus Al, Au, AuCu₃, ITO, TiAu, AlAu, Graphen, LaB₆, GdE₆, Ni, CO, Fe, Pd, Pt sowie den Legierungen dieser unterschiedlichen Materialien, und dass eine Schicht wenigstens eines Typs von Molekülen der folgenden Formel I-1: 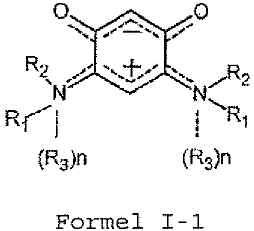 in der
- n = 0 oder 1 und
- R₁ und R₂ aus den folgenden Paaren ausgewählt sind, R₁ ist 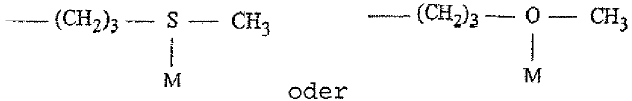 wobei in diesem Fall R₂ H ist und n = 0 ist,
R₁ ist: -C₄H₉, wobei in diesem Fall:
entweder R₂ H ist und n = 1, wobei in diesem Fall R₃ M ist,
oder R₂ M ist und n = 0
R₁ ist -CH₂-C₆H₅-M, wobei in diesem Fall R₂ H ist und n = 0 ist,
R₁ ist -CH₂-C₆H₅, wobei in diesem Fall:
entweder R₂ H ist und n = 1 ist, wobei in diesem Fall R₃ M ist,
oder R₂ M ist und n =0 ist,
wobei M ein Atom oder ein Molekül des Materials bezeichnet, aus dem die Oberfläche des Trägers besteht,
an die genannte Oberfläche gebunden ist.

9. Substrat gemäß Anspruch 8, **dadurch gekennzeichnet, dass** in der Formel I-1 R ausgewählt ist aus einer Gruppe -CH₂-C₆H₅-M und einer Gruppe: 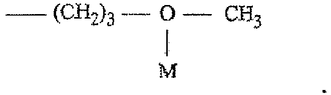 wobei M ein Atom oder ein Molekül des Materials bezeichnet, aus dem die Oberfläche des Trägers besteht

10. Vorrichtung, die ein Substrat umfasst, das Löcher- oder Elektronentransporteigenschaften hat, **dadurch gekennzeichnet, dass** das Substrat einen Träger umfasst, von dem wenigstens eine Oberfläche aus einem leitenden Material ist, und dass eine Schicht wenigstens eines p-Benzochinonimin-Derivats der folgenden Formel I: 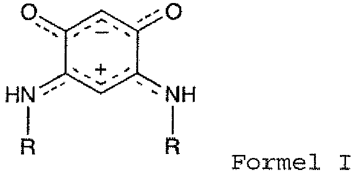 in der R darstellt:
- H,
- einen linearen oder verzweigten C₁-C₂₀-Alkylrest, der mit einem Rest oder mehreren Resten, ausgewählt aus den Resten Hydroxy, Amino, C₁-C₁₂-Alkylamino, C₁-C₁₂-Alkoxy, Pyridin, Phosphin, Thioether, Thiol, C₁-C₁₂-Alken, C₁-C₁₂-Alkin und Halogen, substituiert sein kann,
- einen Benzylrest, der mit einem Rest oder mehreren Resten, ausgewählt aus den Resten Hydroxy, Amino, C₁-C₁₂-Aminoalkyl, C₁-C₁₂-Alkoxy und Halogen, substituiert sein kann.
an dieses leitende Material gebunden ist.

11. Vorrichtung gemäß Anspruch 10, **dadurch gekennzeichnet, dass** in der Formel I R ausgewählt ist aus einem Wasserstoffatom oder einer Gruppe -C₄H₉, -C₃H₆-S-CH₃, C₆H₅-CH₂- und -C₃H₆-O-CH₃.

12. Vorrichtung gemäß Anspruch 10 oder 11, **dadurch gekennzeichnet, dass** in der Formel I R aus einer Gruppe -C₄H₉ und einer Gruppe C₆H₅-CH₂- ausgewählt ist.

13. Vorrichtung gemäß einem der Ansprüche 10 bis 12, **dadurch gekennzeichnet, dass** das leitende Material aus einem Material ist, das ausgewählt ist aus Al, Au, AuCu₃, ITO, TiAu, AlAu, Graphen, LaB₆, GdB₆, Ni, CO, Fe, Pd, Pt sowie den Legierungen dieser unterschiedlichen Materialien.

14. Vorrichtung gemäß Anspruch 10, **dadurch gekennzeichnet, dass** das leitende Material aus Al, Au, AuCu₃, ITO, TiAu, AlAu, Graphen, LaB₆, GdB₆, Ni, CO, Fe, Pd, Pt sowie den Legierungen dieser unterschiedlichen Materialien ausgewählt ist, und dadurch, dass das wenigstens eine p-Benzochinonimin-Derivat ein Molekül der folgenden Formel I-1 ist: 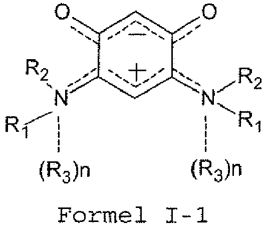 in der
- n = 0 oder 1 und
- R₁ und R₂ aus den folgenden Paaren ausgewählt sind, R₁ ist 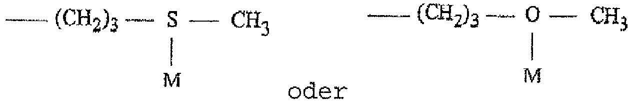
wobei in diesem Fall R₂ H ist und n = 0 ist,
R₁ ist: -C₄H₉, wobei in diesem Fall:
entweder R₂ H ist und n = 1, wobei in diesem Fall R₃ M ist,
oder R₂ M ist und n = 0
R₁ ist -CH₂-C₆H₅-M, wobei in diesem Fall R₂ H ist und n = 0 ist,
R₁ ist -CH₂-C₆H₅, wobei in diesem Fall:
entweder R₂ H ist und n = 1 ist, wobei in diesem Fall R₃ M ist,
oder R₂ M ist und n =0 ist,
wobei M ein Atom oder ein Molekül des leitenden Materials bezeichnet.

15. Vorrichtung gemäß Anspruch 14, **dadurch gekennzeichnet, dass** in der Formel I-1 R₁ ausgewählt ist aus einer Gruppe-CH₂-C₆H₅-M und einer Gruppe: 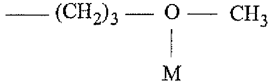 wobei M ein Atom oder ein Molekül des leitenden Materials bezeichnet.

16. Vorrichtung gemäß einem der Ansprüche 10 bis 15, **dadurch gekennzeichnet, dass** es sich um eine Vorrichtung des Typs OLED handelt.

17. Vorrichtung gemäß einem der Ansprüche 10 bis 15, **dadurch gekennzeichnet, dass** es sich um eine Vorrichtung des Typs OPV handelt.

18. Vorrichtung gemäß einem der Ansprüche 10 bis 12, **dadurch gekennzeichnet, dass** es sich um eine Vorrichtung des Typs OFET handelt.

## Claims

1. Use of p-benzoquinonemonoimine derivatives of the following formula I: 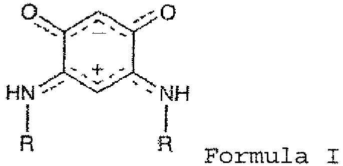 wherein R represents:
- H,
- a linear or branched C₁ to C₂₀-alkyl radical which can be substituted by one or more radicals chosen from the radicals hydroxy, amino, C₁ to C₁₂ alkylamino, C₁ to C₁₂ alkoxy, pyridine, phosphine, thioether, thiol, C₁ to C₁₂ alkene, C₁ to C₁₂ alkyne and halogen,
- a benzyl radical which can be substituted by one or more radicals chosen from the radicals hydroxy, amino, C₁ to C₁₂ alkylamino, C₁ to C₁₂ alkoxy and halogen,
for forming a hole or electron transport layer.

2. Use according to claim 1, **characterised in that**, in formula I, R is chosen among a hydrogen atom or a group -C₄H₉, -C₃H₆-S-CH₃, C₆H₅-CH₂- and -C₃H₆-O-CH₃.

3. Use according to claim 1 or 2, **characterised in that**, in formula I, R is chosen from a group -C₄H₉ and a group C₆H₅-CH₂-.

4. Process for the production of a substrate having hole or electron transport properties, **characterised in that** it comprises bonding, to at least one surface made of a conducting material of a support, at least one p-benzoquinoneimine derivative of the following formula I: 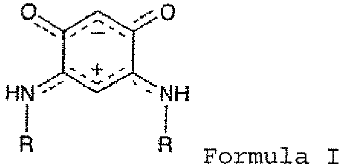 wherein R represents:
- H,
- a linear or branched C₁ to C₂₀ alkyl radical which can be substituted by one or more radicals chosen from the radicals hydroxy, amino, C₁ to C₁₂ alkylamino, C₁ to C₁₂ alkoxy, pyridine, phosphine, thioether, thiol, C₁ to C₁₂ alkene, C₁ to C₁₂ alkyne and halogen,
- a benzyl radical which can be substituted by one or more radicals chosen from the radicals hydroxy, amino, C₁ to C₁₂ alkylamino, C₁ to C₁₂ alkoxy and halogen.

5. Process according to claim 4, **characterised in that**, in formula I, R is chosen from a hydrogen atom or a group -C₄H₉, -C₃H₆-S-CH₃, C₆H₅-CH₂- and -C₃H₆-O-CH₃.

6. Process according to claim 4 or 5, **characterised in that**, in formula I, R is chosen from a group -C₄H₉ and a group C₆H₅-CH₂-.

7. Process according to anyone of claims 4 to 6, **characterised in that** the surface is made of a material chosen from Al, Au, AuCu₃, ITO, TiAu, AlAu, graphene, LaB₆, GdB₆, Ni, Co, Fe, Pd, Pt, and alloys of these various materials.

8. Substrate having hole or electron transport properties, **characterised in that** it comprises a support of which at least one surface is made of a material chosen from Al, Au, AuCu₃, ITO, TiAu, AlAu, graphene, LaB₆, GdB₆, Ni, Co, Fe, Pd, Pt, and alloys of these various materials, and **in that** there is bonded to said surface a layer of at least one type of molecule of the following formula I-1: 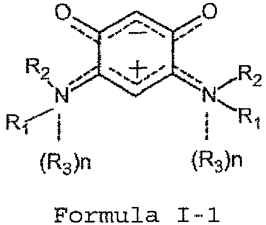 wherein:
- n = 0 or 1, and
- R¹ and R² are selected from the following pairs:
∘ R¹ is 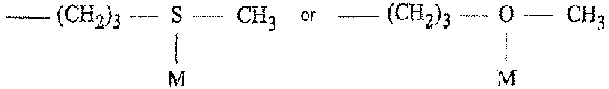 in which case R² is H and n = 0
o R¹ is: -C₄H₉, in which case:
• either R² is H and n = 1, in which case R³ is M
• or R² is M and n = 0
∘ R¹ is -CH₂-C₆H₅-M, in which case R² is H and n = 0
∘ R¹ is -CH₂-C₆H₅, in which case:
• either R² is H and n = 1, in which case R³ is M
• or R² is M and n = 0,
wherein M denotes an atom or a molecule of the material of which said surface of the support is composed,
is bonded to said surface.

9. Substrate according to claim 8, **characterised in that**, in formula I-1, R₁ is chosen from a group -CH₂-C₆H₅-M and a group: 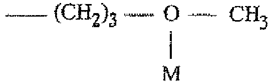 wherein M designates an atom or a molecule of material of which said surface of the support is composed.

10. Device comprising a substrate having hole or electron transport properties, **characterised in that** the substrate comprises a support at least one surface of which is made of a conducting material and **in that** a layer of at least one *p*-benzoquinoneimine derivative of the following formula I, 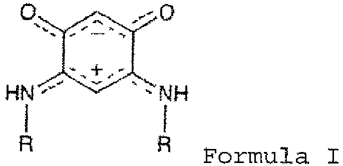 wherein R represents:
- H,
- a linear or branched C₁ to C₂₀ alkyl radical which can be substituted by one or more radicals chosen from the radicals hydroxy, amino, C₁ to C₁₂ alkylamino, C₁ to C₁₂ alkoxy, pyridine, phosphine, thioether, thiol, C₁ to C₁₂ alkene, C₁ to C₁₂ alkyne and halogen,
- a benzyl radical which can be substituted by one or more radicals chosen from the radicals hydroxy, amino, C₁- to C₁₂-alkylamino, C₁- to C₁₂-alkoxy and halogen
is linked to this conductive material.

11. Device according to claim 10, **characterised in that**, in formula I, R is chosen from a group -C₄H₉, -C₃H₆-S-CH₃, C₆H₅-CH₂- and -C₃H₆-O-CH₃.

12. Device according to claim 10 or 11, **characterised in that**, in formula I, R is chosen from a group -C₄H₉ and a group C₆H₅-CH₂-.

13. Device according to any of claims 10 to 12, **characterised in that** the conductive material is a material chosen from Al, Au, AuCu₃, ITO, TiAu, AlAu, graphene, LaB₆, GdB₆, Ni, Co, Fe, Pd, Pt, and alloys of these various materials.

14. Device according to claim 10, **characterised in that** the conductive material is chosen from Al, Au, AuCu₃, ITO, TiAu, AlAu, graphene, LaB₆, GdB₆, Ni, Co, Fe, Pd, Pt, and alloys of these various materials, and **in that** the at least one p-benzoquinoneimine derivative is a molecule of the following formula I-1: 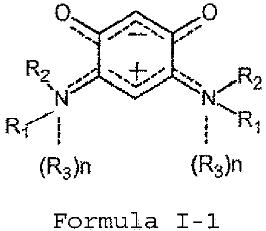 wherein:
- n = 0 or 1, and
- R¹ and R² are selected from the following pairs:
∘ R¹ is 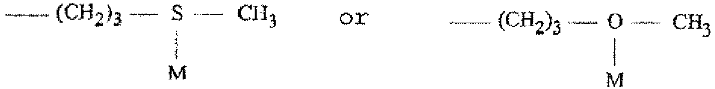 in which case R² is H and n = 0
o R¹ is: -C₄H₉, in which case:
• either R² is H and n = 1, in which case R³ is M
• or R² is M and n = 0
∘ R¹ is -CH₂-C₆H₅-M, in which case R² is H and n = 0
o R¹ is -CH₂-C₆H₅, in which case:
• either R² is H and n = 1, in which case R³ is M
• or R² is M and n = 0,
wherein M denotes an atom or a molecule of the conductive material.

15. Device according to claim 14, **characterised in that**, in formula I-1, R¹ is chosen from a group -CH₂-C₆H₅-M and a group: 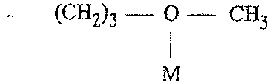 wherein M designates an atom or a molecule of the conductive material.

16. Device according to anyone of claims 10 to 15, **characterised in that** it is a device of the 0LED type.

17. Device according to anyone of claims 10 to 15, **characterised in that** it is a device of the OPV type.

18. Device according to anyone of claims 10 to 15, **characterised in that** it is a device of the OFET type.
